# EUROPEAN PATENT APPLICATION

(11) **EP 0 971 034 A1**
(43) Date of publication of application: **12.01.2000**
(21) Application number: 97912489.8
(22) Date of filing: 18.11.1997
(51) Int. Cl.: C12N 15/13, C12P 21/08, C12N 5/04, C07K 16/00

(54) **PROCESS FOR PREPARING ANTIBODY MOLECULES**

(30) Priority: 19.11.1996 JP 32211696
(71) Applicant: HAGIWARA, Yoshihide, Takarazuka-shi, Hyogo 665 (JP)
(72) Inventor: HAGIWARA, Hideaki, Hyogo 665 (JP); TAKESHIMA, Yasunobu-Haitsu Kakogawa Ichibankan 404, Hyogo665 (JP)
(74) Representative: Weisert, Annekäte, Dipl.-Ing. Dr.-Ing.
(86) International application number: JP9704188
(87) International publication number: WO9822590

(57) **Abstract**

A process for preparing antibody molecules, characterized by transforming cells of blue-green algae with a carrier DNA containing an antibody heavy-chain gene and/or an antibody light-chain gene to separately or simultaneously express an antibody heavy-chain protein and an antibody light-chain protein with the cells, and associating the antibody heavy-chain protein with the antibody light-chain protein *in vitro* or *in vivo*. The process enables antibody molecules having a desired activity to be prepared with a high efficiency.

## Description

### Technical Field

This invention relates to a method for preparing an antibody molecule, more specifically to a method for swift and efficient production of an antibody molecule of desired activity by use of cyanobacteria cells (blue-green algae).

### Background Art

The development of production techniques for monoclonal antibodies using cell fusion or immortalization of cells and the like has made it possible to obtain useful antibodies from human, mouse and the like. A large number of genes which encode these antibodies have been cloned from antibody producing cells, and it has become possible to artificially recombinate on the genetic level regions of the antibody molecule (CDR: Complementarity-Determining Region) that are related to the binding with the antigen.

However, it is still not easy to obtain a specific antibody molecule which binds to a particular antigen. In order to examine by modeling study by use of computer graphics what kind of reactivities an antibody molecule which has been modified in its CDR region actually exhibits towards the antigen of interest, it is necessary to express the antibody molecule in some kind of cell. The construction of a system which uses cultured animal cells as a host to express antibody molecules is rather complicated, moreover such a system is not always able to express the antibody molecule in an efficient manner.

The object of present invention is to provide a new method for solving these kinds of problems. In other words, the main object of present invention is to obtain an antibody molecule of a desired activity in a swift manner by constructing a plasmid or a carrier DNA into which a gene of the heavy chain or the light chain of an antibody of a human or other animal such as mouse and the like has been integrated, and to introduce these DNAs into cyanobacteria cells for expressing the heavy chain and the light chain separately or simultaneously, and then to activate them *in vitro* or *in* *vivo*.

### Disclosure of the Invention

The present invention is to provide a method for preparing an antibody molecule characterized in that each of the antibody heavy chain protein and the antibody light chain protein is individually expressed in cyanobacteria cells by transforming cyanobacteria cells with a carrier DNA which contains the antibody heavy chain gene or the antibody light chain gene, followed by *in vitro* association of the antibody heavy chain protein and the antibody light chain protein.

The present invention furthermore provides a method for preparing an antibody molecule characterized in that each of the antibody heavy chain protein and the antibody light chain protein is individually expressed in cyanobacteria cells by transforming cyanobacteria cells with a carrier DNA which contains the antibody heavy chain gene and the antibody light chain gene, followed by *in vivo* association of these proteins.

In other words, the present invention is to prepare an active antibody molecule by using as a host cyanobacteria cells which grow faster than cultured animal cells and which allow easy and efficient expression of heterogeneous protein by linking a gene which encodes the heavy chain and/or the light chain of the antibody to a promoter, a terminator and the like, all of which show activity in the host cells, and by using these as a carrier DNA or using a plasmid into which these are introduced and which can replicate in the host cells, for transforming the cyanobacteria cells to efficiently express the heavy chain and light chain proteins of the antibody in the host cells, followed by associating said proteins *in vitro* or *in vivo*. This method allows screening of an antibody molecule of a desired activity in a much faster, efficient and economic manner then when using an animal cell culture system.

### Brief Description of the Drawing

Figure 1 is a construction scheme of an expression cassette for simultaneous expression of the heavy chain gene and the light chain gene of monoclonal antibody CLN-IgG according to present invention.
Figure 2 is the base sequence of the linker DNA used in example 4 hereinafter.
Figure 3 is the base sequence of the spacer DNA used in example 4 hereinafter.

The method of present invention will be explained in further detail hereinafter.

### (1) Creation of the carrier DNA

The carrier DNA which can be used to transform cyanobacteria cells according to present invention, can be created by linking the structural gene encoding the heavy chain and/or the structural gene encoding the light chain of an antibody of a human or other mammalian such as mouse and the like to a transcription initiation region showing promoter activity in the host cell, a SD-like sequence, a transcription termination region, a spacer region and the like.

### ① Structural gene encoding the heavy chain or the light chain of the antibody

There are no specific limitations for the antibody genes which can be expressed according to the method of present invention, allowing to use structural genes which encode the heavy chain or the light chain of any antibody, or a gene which encodes the amino acid sequence of a part of the structural gene. Moreover, although the antibody gene can be cDNA, RNA, or synthetic DNA, in general cDNA is the most suitable. Specific examples of said antibody gene, are for example, cDNA, RNA, synthetic DNA or the like which encode the heavy chain or the light chain of human monoclonal antibody CLN-IgG which is described in Japanese Laid-Open Patent Publication No. 121859/1997 and PCT International Publication WO 92/20799. These antibody genes can be isolated from a plasmid and the like in which they are contained by one of the standard methods.

### ② Transcription initiation region

For creating the carrier DNA a transcription initiation (promoter) region that is suitable to the kind of cyanobacteria used as a host is selected. Specific examples for such promoter regions are for example, the promoter region of ribulose-1,5-bisphosphate carboxylase / oxygenase gene [K. Shinozaki *et al*., Proc. Natl. Acad. Sci. USA, 80: 4050-4054 (1983)], promoter I and II of Cyanophage N1 [G. J. Schneider *et al*., Gene 105: 51-60 (1991)], the promoter region of *psbA* gene of *Anabaena* PCC7120 [J. Elhai, FEMS Microbiol. Letter, 114: 179-184 (1993)], the promoter region of *RuBisCO* gene of *Anacystis nidulans* which can be prepared by cutting out plasmid pANE18 (a plasmid with an inserted EcoRI fragment of approximately 5.6 MDa, which contains the RuBisCO promoter region at the EcoRI site of pBR322) described in the publication of K. Shinozaki *et al*. [Proc. Natl. Acad. Sci. USA, 80: 4050-4054 (1983)] using restriction enzymes *Eco*RI, *Sac*I and *Pst*I according to a conventional method (for example, see Molecular Cloning - A Laboratory Manual - Cold Spring Harbor Laboratory, by T. Maniatis *et al*.).

### ③ SD-like sequence

In the carrier DNA used in the present invention, a SD-like sequence is introduced as ribosome recognition sequence downstream of the promoter region and upstream of each antibody gene. It is preferable to use a SD-like sequence that is complementary to the ribosomal RNA of the host cyanobacteria. In case of using cyanobacterium *Anacystis nidulans* 6301 strain as a host, it is possible to use a GGAGG or GGAG base sequence, but other base sequences which are known as SD-like sequences can be used as well. As such SD-like sequences consist only of few bases, they are often prepared by synthesis.

In addition, usually the SD-like sequence is located ahead of ATG (translation initiation site). As the length (the number of bases) from the SD-like sequence to ATG possibly affects the expression of the antibody gene, it is preferable to adjust it in accordance with the type of the host the base sequences of the antibody gene and the like in order to optimize the amount of expression. Although the length differs depending on the type of cyanobacterium that is used as a host, the base sequences of the antibody genes and the like, it is in general approximately 3 to 10 bases and the optimal length can be empirically determined in accordance with the base sequences of the antibody genes and the like.

Preparation of the DNA fragment which contains the SD-like sequence in general can be conducted up to before ATG (translation initiation site) or even can cover ATG, and the synthesis of the DNA fragment can be easily conducted by genetic manipulation techniques known *per se* [for example, see "Biochemical Experiments Series Cours 1 Genetic Research Methods II" edited by Japanese Biochemical Academy, published by Tokyo Kagaku Dohjin (1997); Molecular Cloning - A Laboratory Manual Second Edition, J. Sambrock *et al*., Cold Spring Harbor Laboratory Press (1988)].

### ④ Transcription termination site

A transcription termination (terminator) region is selected which can be used for creating a carrier DNA and which is suitable to the kind of cyanobacterium that is used as a host. Specifically, for example, the terminator region of the *psb*A gene of *Anabaena* PCC7120 [S. E. Curtis and R. Haselkorn, Plant Mol. Biol., 3: 249-258 (1984)], the terminator region of the *SigA* gene [D. Borthakur and R. Haselkorn, Plant Mol. Biol., 13: 427-439 (1989)], the terminator region of the *RuBisCO* gene of *A*. *Nidulans*, which can be prepared by cutting out plasmid pANP1155 (a plasmid with an inserted *Pst*I fragment of approximately 1.5 MDa containing the terminator of *RuBisCO* at *Pst*I site of pBR322) as described in the publication of K. Shinozaki *et al*. [Proc. Natl. Acad. Sci. USA, 80: 4050-4054 (1983)] by a conventional method using restriction enzymes *Eco*RI and *Pst*Iby.

### ⑤ Spacer region

In case of expressing the antibody heavy chain gene and the antibody light chain gene simultaneously in the present invention, in order to express the antibody heavy chain gene and the antibody light chain gene as one single operon, a spacer region should be placed between the antibody heavy chain gene and the antibody light chain gene.

As said spacer region, specifically, the spacer region which is located between a large subunit gene and a small subunit gene which lie on the ribulose 1,5-bisphosphate carboxylase / oxygenase operon [K. Shinozaki *et al*., Proc. Natl. Acad. Sci., USA, 80: 4050-4054 (1983)] , can be given as examples, but it is not limited to this example, namely as long as it is a sequence which is located between genes within said operon, which transcribes more than two genes as a single operon, this sequence can be used as well. Such a spacer region can be isolated from naturally occurring material or can be synthetically generated.

### ⑥ Preparation of an expressible operon which contains the antibody gene

By appropriate linkage of the promoter region, the DNA fragment containing the SD-like sequence, and the terminator and spacer regions prepared as described above to a structural gene encoding the antibody gene, an operon which is expressible in cyanobacteria cells can be created.

For example, by linkage of each of the above-mentioned components in the order of (promoter region)- (DNA fragment containing the SD-like sequence)-(translation initiation codon, ATG)-(antibody heavy chain gene or light chain gene)-(translation termination codon)- (terminator region) by a method known *per se*, an operon which can express the antibody heavy chain or light chain protein in cyanobacteria cells can be created.

Moreover, for example, by linkage of each of the above-mentioned components in the order of (promoter region)-(DNA fragment containing the SD-like sequence)- (translation initiation codon, ATG)-(antibody heavy chain gene)-(translation termination codon)-(spacer region)-(DNA fragment containing the SD-like sequence)- (translation initiation codon, ATG)-(antibody light chain gene)-(translation termination codon)-(terminator region) by a method known *per se*, an operon which can simultaneously express the antibody heavy chain or light chain protein in cyanobacteria cells can be created.

Promoter and SD-like sequences do not have to be single, but it is also possible to use two or more promoters which can be used in tandem, and/or before the SD-like sequence for the antibody light chain gene, a promoter can be placed in tandem, and/or two or more SD-like sequences can be used in tandem.

Although the operon which contains the above-mentioned antibody genes, substantially can transform the host depending on the type(s) of the host and/or the operon, usually the operon is introduced into a vector (plasmid) which is suitable to the host and is used for transformation.

In addition, if an expression plasmid such as pARUB19 [Takeshima *et al*., DNA Res., 1: 181-189 (1994), has (promoter region)-(DNA fragment containing the SD-like sequence)-(multicloning region)-(terminator region)] which was created by the present inventors using a promoter region and a terminator region which show activity in the host cell is used, by introducing the genes which encode the heavy chain or the light chain of the antibody into the multicloning region, for example, *Sph*I site, it is possible to construct an operon which contains the antibody heavy chain or light chain gene and is expressible in cyanobacteria cells.

### ⑦ Vector

As a vector into which said expressible operon can be introduced, a wide variety of vectors which are used in cyanobacteria cells can be used, such as for example pUC104, pUC105 [see, C. J. Kuhlemeier *et al*., Mol. Gen. Genet. 184: 249-254 (1981)]; pLS103 [see, L. A. Sherman and P. van de Putte, J. Bacteriol., 150: 410 -413 (1982)]; pDPL13 [see, S. Gendel *et al*., J. Bacteriol., 156: 148-154 (1983)]; pUC303 [see, C. J. Kuhlemeier *et al*., Plasmid 10: 156-163 (1983)]; pSG111 [see, S. S. Golden and L. A. Sherman, J. Bacteriol., 155: 966-972 (1983)]; pPUC29 [see, C. J. Kuhlemeier *et al*., Gene 31: 109-116 (1984)]; pPLANBal [see, D. E. Laudenbach *et al*., Mol. Gen. Genet., 199: 300-305 (1985)]; pBAS18 [see, K. Shinozaki *et al*., Gene 19: 221 -224 (1982)].

In addition, such a vector can be derived as required, from a plasmid or a virus, and for example, a shuttle vector such as pBAS10, pBAS10R, pBAX18, pBAX18R [see, Takeshima *et al*, DNA Res., 1: 181-189 (1984)]; pBAX20 [see, Japanese Laid-Open Patent Publication No. 281793/1992] which are replicable in *Escherichia coli* and cyanobacteria cells, and which were created by the present inventors from a replication initiation region (*OriA*) of plasmid pBA1 derived from *Anacystis nidulans*, a multicloning region of plasmid pUC and a replication initiation region (*OriE*) of a plasmid of *E. coli* ColE1 system can also be advantageously used.

### ⑧ Construction of a carrier DNA

The expressible operon which contains the antibody genes as mentioned in ⑥ above can be introduced into a vector such as those described in above ⑦ according to a gene manipulation technique known *per se*.

For example, the operon which can be prepared using a structural gene encoding the heavy chain and/or the light chain of monoclonal antibody CLN-IgG as described in said ⑥ is inserted into shuttle vector pBAX18, pBAS10, or pBAX20 which are replicable in the cells of *Anacystis nidulans* by cleaving the operon using for example *Eco*RI, mixing each of the DNA fragments and subjecting to reaction with T4 DNA ligase to obtain a plasmid which expresses the antibody gene. The number of the operon(s) which is/are inserted into the vector does not have to be one, and as long as they are inserted in the same direction, two, three, four or more operons can be placed in tandem.

### (2) Transformation and expression

According to present invention cyanobacteria cells are transformed using the carrier DNA which can be created as described above. Cyanobacteria cells which can be used for this purpose, are for example, *Anacystis nidulans* 6301 strain (*Synechococcus* PCC6301), *Anacystis nidulans* R2 strain (*Synechococcus* PCC7942), *Synechococcus* PCC7002, *Synechococcus* PCC7418 (*Aphanothece halophytica*), *Synechocystis* PCC6803, *Synechoccystis* PCC6714, *Spirulina platensis*, *Anabaena* PCC7120 (*Nostoc* PCC7120), *Nostoc* PCC7119 (*Anabaena* PCC7119), *Calothrix* PCC7601 and the like, as well as a monocytic cyanobacterium which present inventors have isolated from *Suizenjinori* (*Aphanothece sacrum*, see, Japanese Patent Application No. 8-191605).

Transformation of these cyanobacteria cells with said carrier DNA can be conducted according to one of the methods which are known *per se*, such as for example a method of R. D. Porter [CRC Critical Reviews in Microbiology 13 (2): 111-132 (1985)], D. A. Lightfoot *et al*. [J. General. Microbiology 134: 1509-1514 (1988)], S. S. Golden *et al*. [J. Bacteriology 158: 36-42 (1984)], H. Daniell *et al*. [Proc. Natl. Acad. Sci. USA 83: 2546-2550 (1986)], T. Matsunaga *et al*. [Appl. Biochem. Biotechnol. 24/25: 151-160 (1990)], Hidio Ochiai [Bioscience and Industry 49: 749-751 (1991)] or the like.

Transformation can be performed for example, by introducing an expression plasmid with the antibody genes into *Anacystis nidulans* by use of the method of D. A. Lightfoot *et al*., After selecting the resulting transformants by means of ampicillin resistance and the like, by means of immunoblotting method, enzyme-linked immunosorbent assay (ELISA) and the like it can be checked if the transformants are those desired.

The transformants thus obtained are cultured in one of the media that are known *per se* to be suitable for growth of the host cells selected. It is preferable to add at that time, an appropriate agent, for example, ampicillin or the like, for selective growth of the transformant, and, if necessary, an agent for inducing expression can be added to the medium, and temperature, light and the like can be changed.

In case that *Anacystis nidulans* is chosen as a host for the transformant, suitable media are BG-11 medium, MA medium and the like, and regarding culture conditions, generally a culture temperature ranging between 10 and 35°C, but preferably between 25 and 30°C is suitable. Moreover, generally a pH of the medium ranging between 7 and 9, and a light intensity ranging between 500 and 5000 lux are suitable. Under these conditions, cultivation can be conducted for about 5-20 days. In addition, stationary or culture can be performed.

Thus the antibody heavy chain protein and/or the antibody light chain protein are/is expressed in the cyanobacteria cells and in case that both of the proteins are expressed simultaneously, they are associated *in vivo* and the antibody molecule is accumulated in the cells.

On the other hand, if the antibody heavy chain protein and the antibody light chain protein are expressed separately in the cyanobacteria cells, the individual fractions which contain the antibody heavy chain protein or the antibody light chain protein obtained from the cultured cyanobacteria cells, are mixed together at a ratio wherein the amounts of the heavy chain protein and the light chain protein become almost the same, and the mixture is incubated at an appropriate temperature, for example at approximately 4 - 40°C, and optionally is dialyzed against an appropriate buffer solution to associate the antibody heavy chain protein and the antibody light chain protein, for the *in vitro* formation of the antibody molecule with the activity of interest.

### (3) Confirmation of in vitro construction of the antibody molecule or in vivo expression of the antibody molecule in the cyanobacteria cells

Confirmation of the antibody molecule can be conducted by a conventional assay technique, for example, enzyme-linked immunosorbent assay (ELISA), where a molecule showing antigenecity towards said antibody molecule is used.

The antibody molecule constructed or expressed in the way described above can be purified and collected using a conventional method, for example, by appropriate combination of physical and biochemical methods such as salting-out, dialysis, ion exchange chromatography, gel filtration chromatography, chromatofocusing, hydrophobic interaction chromatography, affinity chromatography, electrophoresis and the like.

The thus generated antibody molecule can be used, for example, for screening of antibodies with the activity of interest. Moreover, if the antibody can be collected in large amounts, it can also be used as primary antibody for immunological methods, such as ELISA and the like. In addition, if it is an anti-cancer human monoclonal antibody such as CLN-IgG, the antibody molecule which is expressed in the cyanobacteria can also be used as an anti-cancer vaccine.

### Examples

The present invention will be furthermore explained in detail in the examples hereinafter. The cloning vectors used in the examples were prepared in the following way.

### Preparation of the cloning vector

*Escherichia coli* JM109 strain having cDNA cloning vector pG611 (see, Japanese Laid-Open Patent Publication No. 121859/1997 and WO 92/20799) which encodes the heavy chain (γ) of human monoclonal antibody CLN-IgG and *Escherichia coli* JM109 strain having cDNA cloning vector pK413 (see, Japanese Laid-Open Patent Publication No. 9-121859 and WO 92/20799) which encodes the light chain (κ) of human monoclonal antibody CLN-IgG were added to 100 ml 2YT medium (trypton 18 g/l, yeast extract 10 g/l, sodium chloride 5 g/l) and shake-cultured overnight at 37°C. These two types of cells were individually collected by centrifugation at 10000 rpm for 10 minutes, suspended in 30 ml SET (20 % sucrose, 50 mM Tris-HCl, 50 mM EDTA, pH 7.6) and then subjected to alkaline-SDS method for preparation of the plasmid DNAs. To each of the cell suspensions, 70 ml of a bacteriolysis mixture solution (1 % SDS, 0.2 N sodium hydroxide) were added and by mild mixing lysis of *Escherichia coli* was performed. After cooling on ice for 15 minutes, 3 M potassium acetate (pH 4.8) was added and by mild mixing the denatured genome DNAs, proteins and SDS were precipitated as white matters. These solutions were centrifuged at 10000 rpm at 4°C for 15 minutes to obtain the supernatants and to each of the obtained supernatants, the equivalent volume of isopropanol was added. These mixtures were centrifuged at 10000 rpm at 4°C for 10 minutes to precipitate the plasmid DNAs. The supernatant liquids were discarded, each of the pellets was lysed in 6 ml TE (10 mM Tris, 1 mM EDTA, pH 8.0), and after adding 2 ml of 8 M LiCl to each solution, incubation in an ice bath was performed. After 4 hours, the resulting RNA precipitates were centrifuged at 15000 rpm at 4°C for 15 minutes to obtain the individual supernatants. To each of the supernatants obtained, two volumes of ethanol were added and centrifugation at 15000 rpm at 4°C was carried out for 15 minutes to collect the precipitates of the plasmid DNAs. Each of the plasmid DNAs was dissolved in 2 ml TE and after the addition of 5 µl of 10 g/ml RNase, incubated at 37°C. After two hours, to each of these solutions, the equivalent volume of phenol : chloroform : isoamyl alcohol (25 : 24 : 1) was added and after well mixing by a vortex mixer, the mixtures were centrifuged at 15000 rpm for 4 hours to obtain the individual aqueous layers (twice). To each of these solutions, the equivalent amount of chloroform : isoamyl alcohol (24 : 1) was added and after well mixing, these mixtures were centrifuged at 15000 rpm for 3 minutes to obtain the individual top layers. To each of these aqueous solutions, 200 µl of 5 M sodium chloride and 2.5 volumes of ethanol were added and well mixed, and after leaving them for 10 minutes, the precipitates of the individual plasmid DNAs were centrifuged at 15000 rpm at 4 °C for 20 minutes and collected. Each of the precipitates were washed with 70 % ethanol and exsiccated in a vacuum desiccator. Each of the DNAs were dissolved in 50 µl TE to be used for the following examples.

### Example 1: Construction of an expression plasmid for the antibody heavy chain or light chain gene

### (1-i) Preparation of the antibody heavy chain or light chain genes

Five µg (20 µl) of cDNA cloning vector pG611 which encodes the heavy chain (γ) of human monoclonal antibody CLN-IgG were placed into a 1.5-ml Eppendorf tube, and 10 µl of 10 × H buffer solution (400 mM Tris-HCl, pH 7.5, 100 mM MgCl₂, 10 mM dithiothreitol, 1000 mM NaCl), 4 µl *Eco*RI (10 units/ µl) and sterile water were added to 100 µl. After the contents of this tube had been subjected to reaction at 37°C for 4 hours, 1/10 volume of 3 M potassium acetate and the 3-fold volume of ethanol were added, and then the mixture was cooled in a freezer at -80 °C. This tube was centrifuged at 15000 rpm for 10 minutes to precipitate the DNA. After the supernatant had been discarded and washed with 70 % ethanol, the DNA was exsiccated in a vacuum desiccator.

The remaining DNA was dissolved in 40 µl of 10 mM Tris - 1 mM EDTA (pH 8.0, TE), loaded on an 1 % agarose gel and subjected to electrophoresis at a voltage of 50 V to separate the plasmid DNA and the antibody heavy chain gene (approximately 1.7 kb). Hereafter this gel was stained with ethidium bromide (EtBr) and the band which contained the fragment with the size of interest was cut out on a transilluminator. This gel was cut into small pieces and placed into a 2-ml Eppendorf tube, to which a saturated sodium iodide solution (included in DNA purification kit GeneClean, manufactured by BIO 101 Inc., USA) of 3 volumes of the gel was added. By incubation at 50°C, the agarose gel which contained the DNA of interest was completely dissolved, and 20 µl of glass milk (included in GeneClean) were added. This tube was incubated in an ice bath for 10 minutes and immediately after incubation centrifuged at 15000 rpm for 30 seconds to precipitate the glass milk which then was suspended in 300 µl of a saturated NaI solution and the suspension was washed by another centrifugation. In the same way three times washing with 500 µl NEW washing solution (included in GeneClean) was carried out After complete removal of the washing solution, the glass milk was suspended in 50 µl of sterile water and incubated at 55°C for 5 minutes. After incubation, centrifugation was performed at 15000 rpm for 3 minutes to obtain the supernatant.

Then, the glass milk was suspended in 50 µl TE, the supernatant was obtained by incubation at 55°C and centrifugation, and pooled with the supernatant obtained in the previous step. To this solution, the equivalent volume of phenol (saturated with TE, pH 8.0) was added, and after well mixing by a vortex mixer, it was centrifuged at 15000 rpm for 3 minutes to obtain the aqueous layer. To this solution, the equivalent volume of chloroform : isoamyl alcohol (24 : 1) was added and well stirred, and centrifuged at 15000 rpm for 3 minutes to obtain the top layer. To this aqueous solution, 3.3 µl of 3 M sodium acetate and 1 µl Ethanol Precipitation Mate (manufactured by NipponGene Inc.) were added and after well stirring, 2-2.5 volumes of ethanol were added and centrifugation at 15000 rpm for 10 minutes was carried out to precipitate the DNA. The precipitate was washed with 70 % ethanol, and the DNA was exsiccated in a vacuum desiccator. The DNA obtained was dissolved in 8 µl of sterile water, to which 1 µl of 10 × reaction solution of DNA blunting kit (manufactured by Takarashuzo Co., Ltd.) was added and the mixture was incubated at 70°C. After 5 minutes, this tube was transferred into an incubator of 37°C, and to the tube 1 µl T₄ DNA polymerase (included in DNA blunting kit) was added after which the mixture was incubated for 5 minutes. Ninety µl of 50 mM Tris - 5 mM EDTA (pH 8.0) were added and by vigorous vortexing, the reaction was terminated. The DNA was harvested by phenol-chloroform (an equivalent amount) extraction, chloroform extraction and ethanol precipitation. The resulting DNA was dissolved in 10 µl TE and used for the following experiment.

Furthermore, 5 µg (20 µl) of cDNA cloning vector pK413 which encodes the light chain (κ) of human monoclonal antibody CLN-IgG were placed into a 1.5-ml Eppendorf tube, and 10 µl of 10 × H buffer solution, 4 µl of *Eco*RI (10 units/ µl) and sterile water were added to 100 µl. After subjecting the contents of this tube to reaction for 4 hours at 37 °C, the DNA was collected through ethanol precipitation, and then loaded on a 1 % agarose gel and subjected to electrophoresis for separating the plasmid DNA and the antibody light chain gene (approximately 1.0 kb). After staining this gel with EtBr, the band which contained the fragment with the size of interest was cut out on a transilluminator, and then the DNA was extracted from the agarose gel by use of GeneClean. The extracted DNA (100 µl) was purified through phenol treatment, chloroform extraction and ethanol precipitation. The DNA obtained was dissolved in 8 µl of sterile water and then both ends of this DNA were blunted using DNA Blunting Kit. The DNA was precipitated by phenol-chloroform (an equivalent volume) extraction, chloroform extraction and ethanol precipitation. The DNA obtained was dissolved in 10 µl of TE and used for the following experiment.

### (1-ii) Preparation of a vector for the construction of an expression plasmid

Five µg of expression vector pARUB19 (Takeshima *et al*., DNA Research 1: 181-189, 1994) for *Anacystis nidulans* 6301 strain (*Anacystis nidulans* 6301) were placed into a 1.5-ml Eppendorf tube, to which 5 µl of 10 × K buffer solution (200 mM Tris - HCl, 100 mM MgCl₂, 10 mM dithiothreitol, 1000 mM KCl, pH 8.5), 2 µl *Sph*I (manufactured by Takarashuzo Co., Ltd., 24 units) and sterile water were added to 50 µl, and this mixture was incubated at 37°C for 4 hours. This reaction solution was separated into undigested vector DNA and *Sph*I-digested vector DNA by electrophoresis using 1 % agarose gel. The *Sph*I-digested vector DNA was extracted from the agarose gel using GeneClean, and purified through phenol extraction, chloroform extraction and ethanol precipitation. The purified DNA was dissolved in 44 µl of sterile water, to which 5 µl of 10 × reaction solution (500 mM Tris- HCl, 10 mM MgCl₂, pH 9.0) and 1 µl (0.3 units/ µl) alkaline phosphatase (derived from *Escherichia coli* C75, manufactured by Takarashuzo Co., Ltd.) were added, and this mixture was incubated at 65°C. After one hour, another 1 µl of the alkaline phosphatase was added after which this mixture was incubated at 37°C for one hour. The DNA was purified and harvested from the reaction solution through phenol-chloroform extraction (an equivalent volume), chloroform extraction and ethanol precipitation. The harvested DNA was dissolved in 20 µl TE and used for the following experiment.

### (1-iii) Construction of the expression plasmid

To 0.1 µg (1 µl) of the *Eco*RI fragment which encoded the heavy chain (γ) of CLN-IgG prepared in (1-i) and (1-ii) described above and 0.4 µg (2 µl) of pARUB19 treated with *Sph*I, polymerase and alkaline phosphatase, solution A (15 µl) of Takarashuzo ligation kit was added and well mixed. After adding solution B (3 µl) and well mixing, the mixture was incubated overnight at 16°C. Ten µl of this reaction solution were added to a competent cell solution of *Escherichia coli* JM109 strain (manufactured by Nippon Gene Inc.: 100 µl), and after gentle stirring this mixture was immediately incubated in an ice bath. After incubation for 30 minutes, the mixture was transferred to a water bath of 37°C for 2 minutes to subject cells to heat shock treatment so that the DNA was incorporated into the cells. After heat shock treatment, the mixture was immediately transferred to an ice bath in which it was left for 5 minutes. Then 1 ml 2YT medium (trypton 18 g/l, yeast extract 10 g/l, sodium chloride 5 g/l) was added and the mixture was incubated at 37°C for one hour under slow shaking. Fifty, 100 and 150 µl of this cell suspension were spread onto 2YT agar medium (containing 50 µg/ml ampicillin and 1.5 % agar).

These plates were incubated overnight at 37°C and several colonies were obtained. The colonies obtained were reinoculated on 1.5 ml 2YT medium (containing 50 µg/ml ampicillin) and shake-cultured overnight at 37°C. Each culture solution was transferred into a 2 ml-Eppendorf tube, which was centrifuged at 10000 rpm for one minute for collecting the cells. After discarding the supernatant, the pellet of the remaining cells was suspended in 150 µl SET (20 % sucrose, 50 mM Tris-HCl, 50 mM EDTA, pH 7.6) and the plasmid DNA was prepared through alkaline - SDS method. To the cell suspension, 350 µl of bacteriolysis mixture solution (1 % SDS, 0.2 N sodium hydroxide) were added and the mixture was gently stirred to lyse *Escherichia coli*. After cooling on ice for 10 minutes, 150 µl of 3 M potassium acetate (pH 4.8) were added and denatured genome DNAs, proteins and SDS were precipitated as white matters. This solution was centrifuged at 15000 rpm for 10 minutes to obtain the supernatant, to which the equivalent volume of isopropanol was added. By centrifugation at 15000 rpm for 10 minutes, the plasmid DNA and RNA were precipitated. The supernatant was discarded and the pellet was dissolved in 100 µl TE, to which 2 µl of RNase (10 mg/ml) were added and the mixture was incubated at 37°C. After two hours, this solution was subjected to phenol : chloroform, chloroform treatment and to the supernatant obtained, 3 volumes of ethanol and 1/10 volume of 3 M sodium acetate (pH 4.8) were added after which this mixture was centrifuged at 15000 rpm for 10 minutes. After discarding the supernatant and washing the pellet with 70 % ethanol, the pellet was exsiccated in a vacuum desiccator.

The resulting pellet of the plasmid DNA was dissolved in 25 µl of sterile water, to which 3 µl of 10 × H buffer solution and 2 µl *Sal*I (12 units/ µl, manufactured by Takarashuzo Co., Ltd.) were added and the mixture was subjected to reaction at 37°C for 4 hours. The resulting *Sal*I-digests of the plasmid DNA were loaded on a 1 % agarose gel and subjected to electrophoresis. As a result, two clones out of 28 cultured clones were found to retain the gene of interest (which encodes the heavy chain (γ) of CLN-IgG) inserted in the expressible direction. One of these two clones was inoculated in 200 ml 2YT medium (containing 100 µg/ml ampicillin) and cultured at 37°C overnight. Cells were collected by centrifugation at 10000 rpm for 10 minutes, the plasmid DNA was extracted from the cells through alkaline-SDS method, and purified through RNase treatment, phenol extraction (twice), chloroform extraction and ethanol precipitation (pARUBH11-γ).

On the other hand, to 0.1 µg (1 µl) of the *Eco*RI fragment which encodes the light chain (κ) of CLN-IgG prepared in (1-i) and (1-ii) described above and 0.4 µg (2 µl) of pARUB19 treated with *Sph*I, polymerase and alkaline phosphatase, solution A (15 µl) of Takarashuzo ligation kit were added and mixed. Then solution B (3 µl) was added and well mixed and the mixture was incubated overnight at 16°C. Competent cells of *Escherichia coli* JM109 strain were transformed with this reaction solution, after which these cells were spread onto plates. After overnight incubation of these plates at 37°C, several colonies were obtained on 2YT agar medium (containing 50 µg/ml ampicillin, 1.5 % agar). The resulting colonies were reinoculated into 1.5 ml 2YT medium (containing 50 µg/ml ampicillin) and cultured overnight at 37°C. Each of the cultured solutions was individually transferred to a 2 ml-Eppendorf tube, which was centrifuged at 10000 rpm for one minute to collect the cells, and the plasmid DNA was extracted through alkaline-SDS method.

Each of the resulting plasmid DNAs was dissolved in 25 µl of sterile water, to which 3 µl of 10 × H buffer solution and 2 µl *Pst*I (12 units/ µl, manufactured by Takarashuzo Co., Ltd.) were added, and the mixture was subjected to reaction at 37°C for 4 hours. The resulting *Pst*I-digests of the plasmid DNA were loaded on a 1 % agarose gel and subjected to electrophoresis. As a result, two out of the cultured 14 clones were found to retain the plasmid inserted in the direction in which the gene of interest (which encodes the light chain (κ) of CLN-IgG) could be expressed. One of these two clones was inoculated in 200 ml 2YT medium (containing 100 µg/ml ampicillin) and cultured overnight at 37°C. Cells were collected by centrifugation at 10000 rpm for 10 minutes, the plasmid DNA was extracted from the cells trough alkaline-SDS method, and purified trough RNase treatment, phenol extraction (twice), chloroform extraction and ethanol precipitation (pARUBH11-κ).

### Example 2: Expression of the antibody heavy chain and light chain genes in Anacystis nidulans 6301 strain

### (2-i) Transformation of Anacystis nidulans 6301 strain with the expression plasmid of the antibody genes

*Anacystis nidulans* 6301 strain (ATCC 27144) was cultured in 100 ml BG-11 liquid medium (see Table 1 described hereinafter) at 30 °C under illumination for 4 days and then centrifuged at 10000 rpm for 8 minutes to collect the cells. The pellet of the cells was suspended in fresh medium and the number of the cells was adjusted to 1 × 10⁹ cells/ml (0.5 OD_{730 nm} = 5 × 10⁸ cells/ml). 0.5 µg (5 µl) each of the expression plasmids pARUBH11-γ and pARUBH11-κ prepared in Example 1, (1-iii) were added to the cell suspension (1 ml) which had been adjusted to 1 × 10⁹ cells/ ml. After gentle stirring, the tubes containing these cells were covered with aluminum foil and cultured at room temperature for one night with a rotary shaker (manufactured by Taiyoh Kagaku Inc.; RT-50). Hereafter tubes were uncovered and incubated under illumination (approximately 2000 lux) for 6 hours. Partial amounts of these cell suspensions (25, 50 and 100 µl) were taken out and spread on BG-11 agar medium (containing 1 µg/ml ampicillin, 1 mM sodium thiosulfate and 1.5 % agar). These plates were incubated under illumination (approximately 2000 lux) at room temperature for 7-10 days and several colonies with ampicillin resistance were obtained.

**Table 1**

| Component | G-11 mg/l |
|---|---|
| NaNO₃ | 1500 |
| K₂HPO₄ | 40 |
| MgSO₄ 7H₂O | 75 |
| CaCl₂ 7H₂O | 36 |
| Na₂CO₃ | 20 |
| H₃BO₃ | 2.86 |
| NnCl₂ 4H₂O | 1.81 |
| ZnSO₄ 7H₂O | 0.222 |
| CuSO₄ 5H₂O | 0.079 |
| Co(NO₃)₂ 6H₂O | 49.4 µg |
| Na₂MoO₄ 2H₂O | 0.39 |
| Ferrous tartrate | 6 |
| EDTA 2Na 2H₂O | 10 |
| Citric acid | 6 |
| pH | 7.1 |

### (2-ii) Quantification of the antibody expressed in the transformant by means of ELISA

Each of the colonies grown on the plates was transferred into 2 ml BG-11 liquid medium (containing 5 µg/ml ampicillin) and cultured under illumination (approximately 2000 lux) at room temperature by use of a rotary shaker for about 10 days. Each of these cultured solutions was transferred into 100 ml of BG-11 liquid medium (containing 50 µg/ml ampicillin) and cultured for two weeks. Hereafter, each culture solution was transferred to a 12 L-spinner containing 10 L BG-11 liquid medium (containing 50 µg/ml ampicillin), and cultured for 20 days (on the 10th day ampicillin was added in amounts to reach a concentration of 50 µg/ml). The cells from each colony were individually collected by centrifugation at 10000 rpm for 10 minutes, and then suspended in 20 ml of PBS (Na₂HPO₄ 5.75 g/l, KH₂PO₄ 1.0 g/l, sodium chloride 40 g/l and potassium chloride 1.0 g/l, pH 6.7-6.8) containing 1 mM PMSF (phenylmethylsulphonyl fluoride). The cells from each colony were individually distracted with ultrasonic shredding equipment (Sonicator Model W-220: manufactured by Heat Systems-Ultrasonic, Inc.) (15 seconds × 5), and centrifuged at 15000 rpm at 4°C for 20 minutes to obtain the individual supernatants.

Each of the cell extraction solutions obtained was used for Enzyme Linked Immunosorbent Assay (ELISA) to examine whether the antibody genes were expressed in the transformed cyanobacteria. Each well of a 96-well plate (manufactured by Nunc Inc.) was filled with 50 µl of the primary antibody solution (each was diluted 100-fold with PBS: for measurement of the heavy chain (γ), affinity purified goat anti-human IgG Fc manufactured by Corganon Teknika N. V. Inc. was used; for measurement of the light chain (κ), goat antiserum directed against human kappa light chain manufactured by kent Laboratories Inc. was used), and then incubated at 37 °C for 30 minutes. After discarding the primary antibody, each well was washed three times with 200 µl gelatin buffer solution (PBS containing 3 % gelatin). Then, each well was filled with 200 µl of PBS containing 1 % BSA and then incubated at 37°C for 30 minutes to perform blocking. After three times washing of each well with 200 µl gelatin buffer solution, 50 µl of diluted cell extract solution were added into each well (10 times, 50 times and 100 times of dilution) and incubation for one hour at 37°C was performed. After each well was washed three times with 200 µl gelatin buffer solution, to each well 50 µl of the secondary antibody (diluted 3000-fold with PBS: for measurement of the heavy chain (γ), peroxydase standard anti-human IgGγ manufactured by The Binding Site LTD was used, for measurement of the light chain (κ), peroxydase standard human kappa light chain manufactured by The Binding Site LTD was used) were added, and then incubation for 30 minutes at 37°C was performed. After each well was washed five times with 200 µl gelatin buffer solution, each well was filled with 30 ml ortho-phenylenediamine solution (a citric acid buffered solution of pH 5.0 containing 12 mg/30 ml ortho-phenylenediamine and 6 µl/30 ml aqueous hydrogen peroxide solution) and incubated at 37°C for 30 minutes in the dark. Immediately after incubation, 50 µl of 5 N sulfuric acid were added to each well to terminate the reaction. Plates where the reaction had been terminated were subjected to colorimetry using two wave lengths, 490 nm and 660 nm, and a microplate reader (MTP-32; manufactured by Corona Denki Co., Ltd.). As a result, although in terms of the expressed amount of the light chain in the cyanobacteria cells, a definite figure could not be obtained because of the weak reactivity of the antibody, the expressed amount of the heavy chain was found to be as high as 1.66 µg (per cell extract solution 1 OD₂₈₀ₙₘ).

### Example 3: In vitro reconstruction of the antibody proteins which have been separately expressed in cyanobacteria

The absorbance rate at 280 nm of each of the extracts [Example 2, (2-ii)] prepared from various cells expressing the heavy chain protein and the light chain protein of monoclonal antibody CNL-IgG was measured, and on the basis of these figures, extracts were mixed in amounts to reach concentration ratios of 1 : 1. This mixed solution (approximately 40 ml) was placed into a dialysis tube and dialyzed against a denaturation solution (containing 50 mM Tris-HCl pH 8.0, 10 mM MgCl₂, 10 mM KCl) for 60 hours. Then this solution was applied onto a Protein A Sepharose column (HiTrap™ protein A, manufactured by Pharmacia LKB Biotechnology Inc.) to bind the antibody molecule (CLN-IgG) which had been completely reconstructed by association of the heavy chain (γ) and the light chain (κ) molecules to Protein A. After extensive washing of the column with the denaturation solution, the antibody molecule which bound to Protein A was eluted with 0.1 M glycin-HCl and 0.5 M sodium chloride (pH 2.7). To the eluted antibody solution, a small amount of 1.0 M Tris-HCl (pH 9.0) was added for neutralization, and then filled into a dialysis tube and dialyzed overnight against PBS. This antibody solution was filled in UltraFree (M.W. 30000, manufactured by Millipore Inc.) and centrifuged to accomplish 10-fold concentration. This solution was used for SDS-polyacrylamide gel electrophoresis according to the method of Laemmli (Nature, 227: 680, 1970) and then subjected to silver staining (manufactured by Nihon BioLad Inc.) for comparing its electrochemical characteristics with those of monoclonal antibody CLN-IgG obtained from the culture supernatant of a human-human hybridoma. As a result, in the lane of the antibody solution obtained from cyanobacteria extract, a band showing the same mobility as that of the light chain of the antibody molecule which does not bind to Protein A was detected.

In addition, in order to check whether the antibody molecule which was reconstructed *in vitro* had activity or not, mouse monoclonal antibody Idio No. 33 (which recognizes the idiotope of CLN-IgG, see, Japanese Laid-Open Patent Publication No. 101999/1995 and U.S. Patent No. 5,589,573) was used as primary antibody for ELISA in the same way as in Example 2, (2-ii). As a result it was found, that in the antibody solution in which the antibody proteins had been reconstructed *in vitro*, activated CLN-IgG with a value of 84.5 ng/0.022 OD₂₈₀ₙₘ (0.38 %) was contained.
The above result shows that the antibody molecule can be activated by in vitro reconstruction of the heavy chain protein and the light chain protein of the antibody which were separately expressed in the cyanobacteria cells.

### Example 4: Construction of the plasmid for in vivo expression of the antibody molecule

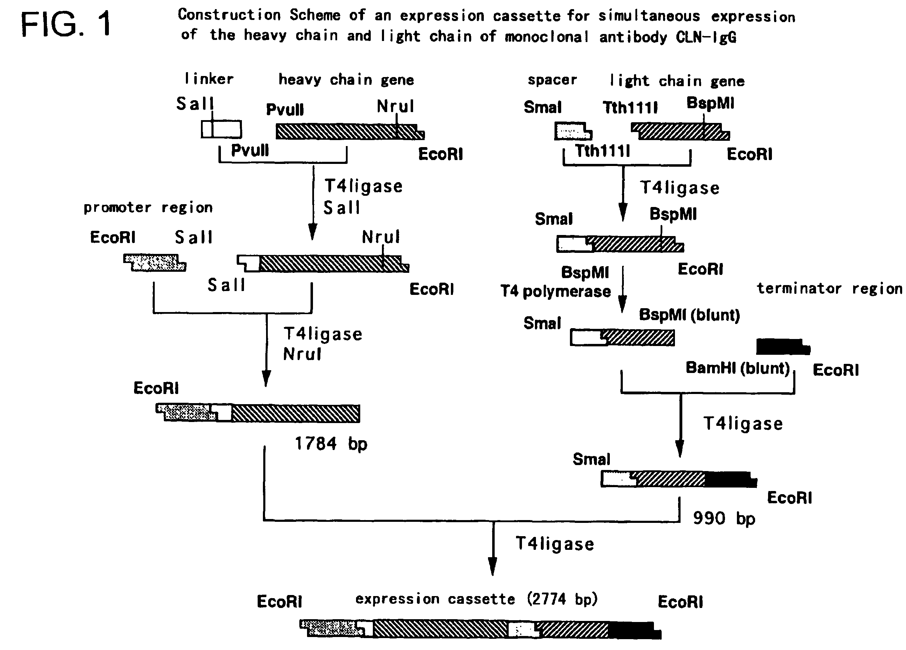

An expression plasmid for monoclonal antibody CLN-H11 was constructed according to the procedures shown in the construction scheme in Figure 1. The procedures will be furthermore explained in detail hereinafter.

### (1-i) Preparation of the antibody heavy chain and light chain genes

The genes which encode the heavy chain (γ) and the light chain (κ) of human monoclonal antibody CLN-IgG were prepared in the same manner as in Example 1, (1-i).

### (1-ii) Preparation of a linker DNA

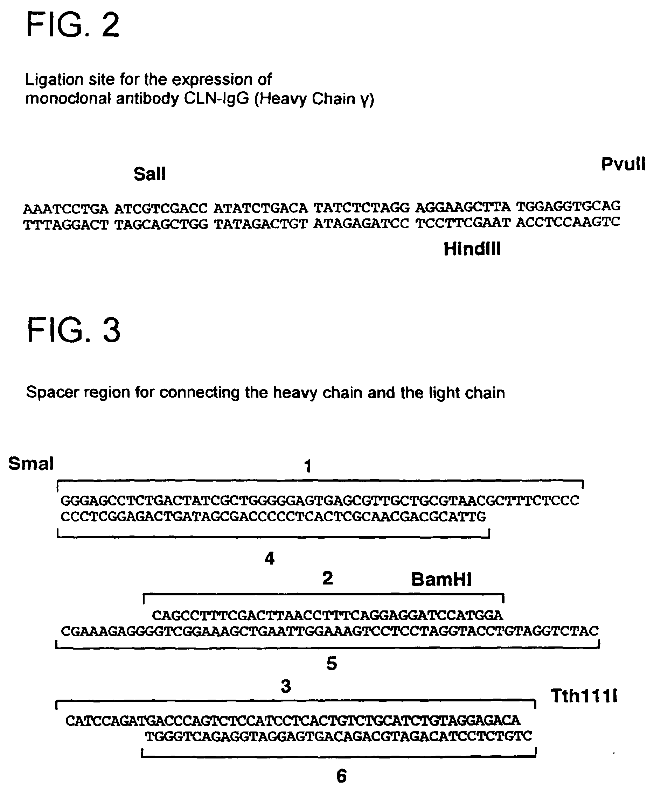

The two oligonucleotides shown in Figure 2 (corresponding to SEQ ID NO: 1) were synthesized by phosphoamidide method using DNA synthesizer 380A (manufactured by Ablied Biosystems Japan Inc.). To the silica gel column in which synthesis had been completed, 0.6 ml of a thiophenol solution (19.5 % thiophenol - 40 % dioxane, - 40 % triethylamine) were added after which the gel was left for more than 8 hours at room-temperature to eliminate the protective group (methyl group) of phosphoric acid. After treatment, in order to eliminate thiophenol, washing was performed by running 4 to 5 times 50 ml methanol through the column. Then, to the column, 2 ml ammonia water (27 % or more) were added in portions of 0.4 ml at intervals of 15 minutes after which the oligonucleotide was cut out from the silica support medium and trapped in a vial. To this vial, another 1 ml ammonia water (27 % or more) was added and after sealing the vial with a cap, parafilm or the like, it was heated at 55°C for more than 8 hours to eliminate the protective group (acyl group) of the base portions. The vial was taken out from the temperature-controlled bath and cooled down to room temperature, before the cap was taken off and the vial was dry-concentrated under reduced pressure. After exciccation, the residue was dissolved in 200 µl of a 0.01 M triethylamine acetate solution (TEAA, pH 7.5) and the main peak portions were fractionated by HPLC using a AM-313-ODS column (manufactured by Yamamura Kagaku Kenkyusho) and acetonitrile - 0.1 M TEAA concentration gradient elution. After dry-concentrating the fractionated main peaks under reduced pressure. 100 µl of 80 % acetic acid (an acetonitrile solution) were added to the concentrate which then was stirred and subjected to room temperature for 30 minutes for eliminating the dimethyl trityl groups (DMTr) at the 5' end and converting them into OH groups. After 30 minutes, swift exsiccation was carried out, and then the residue was dissolved in 200 µl of 0.01 M TEAA (pH 7.5), to which the equivalent volume of diethyl ether was added to extract and eliminate the DMTr groups. After this solution was dry-concentrated under reduced pressure, it was dissolved in 110 µl of 0.01 M TEAA (pH 7.5), and again fractionated and purified by means of HPLC. The solution containing the fractionated oligonucleotide was exsiccated under reduced pressure, after which it was dissolved in 10 mM Tris-HCl - 1 mM EDTA solution A (TE, pH 8.0).

Among the thus synthesized 2 oligonucleotides, the base sequences of which are shown above, 4 µg of the oligonucleotide described in the second line were mixed with 120 µl of a kinase buffer solution (50 mM Tris-HCl, 10 mM MgCl, 0.1 mM EDTA, 5 mM DTT, 0.1 mM spermidine, 1.7 µM ATP, pH 7.6). To this solution, 9 units of T₄ polynucleotide kinase (manufactured by Takarashuzo Co., Ltd.) were added and then incubation at 37°C was performed. After 15 minutes, to this reaction solution, ATP was added to a final concentration of 1 mM, and again 9 units of the T₄ polynucleotide kinase were added, after which this mixture was incubated at 37°C for 25 minutes. After completion of the reaction, the kinase was inactivated by heat treatment at 90°C for 5 minutes. The phosphorylated oligonucleotide was harvested through ethanol precipitation.

To 1 µg of the phosphorylated DNA and 1 µg of the other oligonucleotide that was synthesized (described above), 20 µl of 5 × ligation buffer solution (250 mM Tris-HCl, 10 mM MgCl₂, pH 7.6) and sterile water were added to 80 µl. After this solution was heated for 5 minutes at 90°C, it was slowly cooled down to 4°C over a period of 2 hours, after which 10 µl each of 100 mM DTT and 10 mM ATP were added. Moreover, 2.5 units of T₄ DNA ligase (manufactured by Takarashuzo Co., Ltd.) were added and this mixture was incubated at 4°C for 15 hours. The reaction solution was treated with the equivalent volume of phenol-chloroform and then the linker DNA was collected by ethanol precipitation for use in the following experiment.

### (1-iii) Ligation of the linker DNA and the antibody heavy chain gene

To 1.5 µg of the antibody heavy chain gene (the *Eco*RI-*Eco*RI fragment) prepared in (1-i) above, 5 µl of 10 × M buffer solution (100 mM Tris-HCl, 100 mM MgCl₂, 10 mM DTT, 500 mM NaCl, pH 7.5), 1 µl of *Pvu*II (12 units/ µl, manufactured by Takarashuzo Co., Ltd.) and sterile water were added to 50 µl, and this mixture was incubated at 37°C for 4 hours. The restriction enzyme digested DNA fragments were collected by ethanol precipitation and the DNA fraction of interest (*Pvu*II-*Eco*RI, approximately 1.7 kb) was separated by loading the DNA fractions on a 1 % agarose gel to conduct electrophoresis. The band containing the fragment with the size of interest was cut out and the DNA was extracted from the agarose by use of GeneClean. The extracted DNA (100 µl) was purified through phenol extraction, chloroform extraction and ethanol precipitation, and then dissolved in 4 µl TE.

To 2 µl of the linker DNA (0.1 µg) which had been purified in (1-ii) above and 4 µl of the above-mentioned antibody heavy chain gene (the *Pvu*II-*Eco*RI fragment, 1.0 µg), 0.48 µl of 5 M NaCl and TE were added to 8 µl (final concentration was 0.3 M NaCl). This DNA solution was mized with 8 µl of Takarashuzo ligation kit solution B and then this mixture was incubated for 2 hours at 26°C. After subjecting to reaction, the solution was treated with phenol-chloroform and then subjected to ethanol precipitation to collect the DNA. The harvested DNA was dissolved in 17 of µl sterile water, and then subjected to reaction at 37°C for 2 hours after adding 2 µl of 10 × H buffer solution, 0.5 µl of *Eco*RI (10 units/µl) and 0.5 µl of *Sal*I (10 units/µl). From this reaction solution, the DNA fragment (approximately 1.7 kb) to which the linker DNA and the antibody heavy chain gene were linked was separated by electrophoresis using 1 % agarose gel. The DNA of interest was eluted from the agarose gel using GeneClean, and then purified through phenol extraction, chloroform extraction and ethanol precipitation. The purified DNA was dissolved in 4 µl of sterile water for use in the following experiment.

### (1-iv) Preparation of the promoter region and its ligation to the SalI-EcoRI fragment (linker-antibody heavy chain gene)

To 10 µl (10 µg) of the DNA solution of plasmid pA Rup1 (see, Japanese Patent Publication No. 67393/1995) for cloning of the promoter region of the *RuBisCO* gene of *A. nidulance* 6301 (ATCC 27144), 10 µl of 10 × H buffer solution and 4 µl of *Eco*RI (10 units/ µl), and sterile water were added to 100 µl, and this mixture was incubated at 37 °C. After 3 hours, the reaction solution was extracted with the equivalent volume of phenol-chloroform and chloroform followed by ethanol precipitation to collect the DNA. The collected DNA was dissolved in 87 µl of sterile water to which 10 µl of 10 × H buffer solution and 3 µl of *Sal*I (10 units/ µl) were added, and this mixture was incubated at 37°C for 3 hours. After completion of the reaction, the DNA was collected through ethanol precipitation, and then the DNA fragment of interest (approximately 380 bp) was separated by conducting electrophoresis using 1.5 % agarose, the DNA was electrically eluted from the gel, and purified through phenol treatment, chloroform treatment and ethanol precipitation.

To 1 µl (0.1 µg) of the prepared promoter region (the *Eco*RI*-Sal*I fragment), 3 µl (0.5 µg) of the *Sal*I-*Eco*RI fragment which had been prepared in the above-mentioned (1-iii), 0.9 µl of 2 M NaCl and TE were added to 6 µl (final concentration of 0.3 M NaCl). After adding 6 µl of Takarashuzo ligation kit solution B and well mixing, the mixture was incubated at 26°C for more than one hour. After completion of the reaction, the solution was treated with the equivalent volume of phenol-chloroform followed by ethanol precipitation to collect the DNA. The collected DNA was dissolved in 44 µl of sterile water, to which 5 µl of 10 × *Nru*l buffer solution (100 mM Tris-HCl, 70 mM MgCl₂, 70 mM 2-mercaptoethanol, 1.5 M NaCl, 0.1 % BSA, pH 7.5) and *Nru*l (8 units/µl) were added, and the mixture was incubated at 37°C. After 3 hours, the DNA was collected through ethanol precipitation, and then the DNA fragment of interest (approximately 1784 bp) was separated by conducting 0.1 % agarose gel electrophoresis. The DNA was eluted from the gel using GeneClean, and then purified through phenol treatment, chloroform treatment and ethanol precipitation.

### (1-v) Preparation of the spacer DNAs

Among the six oligonucleotides (or DNAs) shown in Figure 3 (corresponding to SEQ ID NO. 2 in Sequence Listing) synthesized in the same manner as described in the above-mentioned (1-ii), 4 µg each of the oligonucleotides other than oligonucleotide No. 1 were individually phosphorylated at the 5' end with a kinase. The phosphorylated oligonucleotides were collected through ethanol precipitation, and to 1 µg each of the collected oligonucleotides No. 2 . No. 6 as well as 1 µg of oligonucleotide No. 1, 20 µl of 5 × ligation buffer solution and sterile water were added to 80 µl. After this solution was heated at 90°C for 5 minutes, it was cooled down to 4°C over a period of 2 hours and 10 µl each of 100 mM DTT and 10 mM ATP were added. Moreover, 2.5 units of T₄ DNA ligase (manufactured by Takarashuzo Co., Ltd.) were added after which this mixture was incubated at 4°C for 15 hours. The reaction solution was treated with an equivalent volume of phenol-chloroform and the DNA was collected through ethanol precipitation.

### (1-vi) Ligation of the spacer DNA and the antibody light chain gene

To 2.5 µg of the *Eco*RI fragment which encodes the light chain (κ) of CLN-IgG purified in (1-i) above, 5 µl of 10 × K buffer solution, 1 µl of Tth111I (8 units/µl, manufactured by Takarashuzo Co., Ltd.) and sterile water were added to 50 µl, and this mixture was incubated at 65°C for 4 hours. The DNA fragment of interest (*Tth*111I-*Eco*RI, approximately 1.0 kb) was loaded onto a 1.5 % agarose gel, separated by conducting electrophoresis and eluted from the gel using GeneClean. The eluted DNA (100 µl) was purified through phenol treatment, chloroform treatment and ethanol precipitation, and then dissolved in 4 µl TE.

To 1.5 µl of the spacer DNA (0.3 µg) prepared in (1-v) above and 4 µl of the above-mentioned antibody light chain gene (the *Tth*111I-*Eco*RI fragment, 2.0 µg), 0.48 µl of 5 M NaCl and TE were added to 8 µl (to a final concentration of 0.3 M NaCl). This DNA solution was mixed with 8 µl of Takara ligation kit solution B and then incubated at 26°C for 2 hours. After completion of the reaction, this solution was treated with the equivalent volume of phenol-chloroform and the DNA was collected through ethanol precipitation. The collected DNA was dissolved in 44 µl of sterile water, to which 5 µl of 10 × *Bsp*MI buffer solution (100 mM Tris-HCl, 100 mM MgCl₂, 1500 mM NaCl, 10 mM DTT, pH 7.9) and 1 µl of *Bsp*MI (10 units/µl) were added, and this mixture was subjected to reaction at 37°C for 4 hours. This reaction solution was subjected to phenol-chloroform treatment and ethanol precipitation to collect the DNA. The DNA obtained was dissolved in 8 µl of sterile water, and after blunting the end of the *Bsp*MI site using Takara blunting kit, the DNA fragment (*Sma*I-blunt ended *Bsp*MI, approximately 0.75 kb) to which the spacer DNA and the antibody light chain gene were linked was separated through electrophoresis by use of an agarose gel. The DNA of interest was eluted from the agarose gel using GeneClean, and purified by conducting phenol treatment, chloroform extraction and ethanol precipitation. The purified DNA was dissolved in 4 µl of sterile water for use in the following experiment.

### (1-vii) Preparation of the terminator region and ligation with the SmaI-BspMI fragment (spacer-antibody light chain gene)

To 5 µl (2.5 µg) of the DNA solution of plasmid pARut13 (see, Japanese Patent Publication No. 67393/1995) for cloning the terminator region of the *RuBisCO* gene of *A. nidulans* 6301 (ATCC 27144), 10 µl of 10 × K buffer solution, 3 µl of *Bam*HI (10 units/µl), and sterile water were added to 100 µl, and the mixture was incubated at 30°C. After 3 hours, the reaction solution was treated with the equivalent volume of phenol-chloroform and chloroform, the DNA was collected through ethanol precipitation, and then the end of the *Bam*HI site of the resulting DNA was blunted using Takara blunting kit. The blunt-ended DNA was purified through phenol treatment and ethanol precipitation according to a conventional method. This DNA was dissolved in 88 µl of sterile water, to which 10 µl of 10 × H buffer solution and 2 µl (10 units/µl) of *Eco*RI were added, and this mixture was incubated at 37°C for 4 hours. After completion of the reaction, the DNA was collected through ethanol precipitation, and the DNA fragment of interest (blunt-ended *Bam*HI-*Eco*RI, approximately 0.3 kb) was loaded on a 1.5 % agarose gel and separated by conducting electrophoresis. The DNA was electrically eluted from the agarose, and then purified through phenol treatment, chloroform extraction and ethanol precipitation.

To 4 µl of the *Sma*I-*Bsp*MI fragment (1.5 µg) prepared in (1-vi) above and 2.5 µl of the terminator region (the *Bam*HI-*Eco*RI fragment 0.5 µg), 2 M NaCl was added to 8 µl (the final concentration was 0.3 M NaCl). To this solution, 8 µl of Takara ligation kit solution B were added, and after gentle stirring, the mixture was incubated at 26°C for 2 hours. After completion of the reaction, this solution was subjected to phenol treatment, chloroform treatment and ethanol precipitation to collect the DNA. The collected DNA was dissolved in 44 µl of sterile water, to which 5 µl of 10 × H buffer solution and 1 µl (10 units/µl) of *Eco*RI were added, and the mixture was incubated at 37°C for 3 hours. From this solution, the DNA was collected through ethanol precipitation, and the DNA fragment with the size of interest (990 bp) was separated by electrophoresis using 1.5 % agarose. The DNA of interest was eluted from the agarose gel using GeneClean, and purified through phenol treatment, chloroform extractionion and ethanol precipitation.

### (1-viii) Construction of the expression cassette

1.5 µg of the *Sma*I-*Eco*RI fragment constructed in (1-vii) above were dissolved in 100 µl of the kinase buffer solution, to which 9 units of T₄ polynucleotide kinase were added, and the mixture was incubated at 37°C. After 15 minutes, 100 mM ATP was added to the final concentration of 1 mM, then again 9 units of T₄ polynucleotide kinase were added, and the mixture was incubated at 37°C for 25 minutes. After completion of the reaction, the reaction solution was treated with equal volumes of phenol-chloroform and chloroform, and the DNA was collected through ethanol precipitation.

To 0.2 µg (2 µl) of the phosphorylated *Sma*I-*Eco*RI fragment and 0.4 µg (2 µl) of the *Eco*RI-*Nru*I fragment constructed in the above-mentioned (1-iv), 2 M NaCl and TE were added to 6 µl (the final concentration was 0.3 M NaCl). To this solution, 6 µl of Takara ligation kit solution B were added, and the mixture was incubated at 26°C for 2 hours. The reaction solution was treated with phenol and chloroform, after which the DNA was collected through ethanol precipitation. The collected DNA was dissolved in 44 µl of sterile water, to which 5 µl of 10 × H buffer solution and 1 µl (10 units/µl) of *Eco*RI were added, and this mixture was incubated at 37°C. After 3 hours, the DNA fragment with the size of interest (2774 bp) was separated by electrophoresis using 1 % agarose gel, and then purified by GeneClean and ethanol precipitation. The purified DNA fragment was dissolved in 5 µl TE for use in the following experiment.

### (1-ix) EcoRI digestion and alkaline phosphatase treatment of pBAX18

To 10 µg (20 µl) of shuffle vector pBAX18 (FERM BP-4639; see Japanese Laid-Open Patent Publication No. 282793/1992) for cyanobacteria and *Escherichia coli*, which was constructed by the present inventors, 10 µl of 10 × H buffer solution, 4 µl (10 units/µl) of *Eco*RI and sterile water were added to 100 µl. This solution was incubated at 37°C for 4 hours. In order to separate *Eco*RI digests from undigested vector DNA, 1 % agarose gel electrophoresis was conducted. The *Eco*RI digests of interest were purified by GeneClean and ethanol precipitation. The purified DNA was dissolved in 100 µl of 0.1 M TrisHCl - 1 mM MgCl₂ (pH 8.0), to which 1 µl (0.3 units/µl) of alkaline phosphatase was added, and then this mixture was incubated at 37°C. After one hour, another one µl of alkaline phosphatase was added, and then this mixture was incubated at 65°C for one hour. The reaction solution was subjected to phenol treatment, chloroform treatment and ethanol precipitation to purify the DNA, which was then dissolved in 20 µl TE.

### (1-x) Creation of CLN-H11 expression plasmid

To 0.4 µg (2 µl) of the plasmid DNA prepared in (1-ix) above and 0.1 µg (1 µl) of CLN-H11 expression cassette constructed in the above-mentioned (1-viii), Takara ligation kit solution A (15 µl) was added and mixed. Moreover, solution B (3 µl) was added and well mixed, before the mixture was incubated overnight at 16°C. This reaction solution was used to transform the competent cells of *Escherichia coli* JM109 strain (manufactured by Takarashuzo Co., Ltd.), and the resulting cells were spread onto plates. These plates were incubated overnight at 37°C and several colonies were obtained on 2YT agar medium (containing 50 µg/ml ampicillin and 1.5 % agar). The colonies obtained were transferred to 1.5 ml of 2YT medium (containing 50 µg/ml ampicillin), and cultured overnight at 37°C. The culture solution was transferred into a 2 ml-Eppendorf tube, centrifuged at 10000 rpm for one minute to collect the cells, and the plasmid DNA was extracted by alkali-SDS method. Each of the plasmid DNAs obtained was dissolved in 25 µl of sterile water, to which 3 µl of 10 × H buffer solution and 2 µl (10 units/µl) *Eco*RI were added, and the mixture was subjected to reaction at 37°C for 4 hours. The obtained *Eco*RI digests of the plasmid DNA were loaded on a 1 % agarose gel and subjected to electrophoresis. As a result, five out of the 16 clones which were cultured were found to retain the CLN-H11 expression cassette (2774 bp). One of these five clones was reinoculated in 200 ml of 2YT medium (containing 100 µg/ml of ampicillin) and incubated overnight at 37°C. The cells were collected by centrifugation at 10000 rpm for 10 minutes, and the plasmid DNA was extracted from the cells by alkali-SDS method and purified through lithium chloride precipitation, RNase treatment, phenol treatment (twice), chloroform extraction and ethanol precipitation (pBAX-CLN-H11).

### Example 5: Expression of antibody CLN-H11 in cyanobacterium Anacystis nidulans 6301 strain

### (2-i) Transformation of Anacystis nidulans 6301 strain with pBAX-CLN-H11

*Anacystis nidulans* 6301 strain (ATCC 27144) was cultured in 100 ml of BG-11 liquid medium (see, Example 2, Table 1 described above) at 30°C under illumination for 4 days, then cells were collected by centrifugation at 10000 rpm for 8 minutes. The cell pellet was suspended in fresh medium and adjusted to a cell number of 1 × 10⁹ cells/ml (0.5 OD₇₃₀ₙₘ = 5 × 10⁸ cells/ml). To the cell suspension (1 ml) that had been adjusted to a cell number of 1 × 10⁹ cells/ml, 0.5 µg (5 µl) of the expression vector pBAX-CLN-H11 prepared in Example 1 were added. After gentle stirring, tubes with these cells were covered with aluminum foil, and cultured overnight at room-temperature using a rotary shaker (manufactured by Taiyoh Kagaku Inc.; RT-50). Hereafter tubes were uncovered and cultured under illumination (approximately 2000 lux) for 6 hours. Then portions of this cell suspension (25, 50 and 100 µl) were spread on BG-11 agar medium (containing 1 µg/ml ampicillin, 1 mM sodium thiosulfate and 1.5 % agar). These plates were incubated under illumination (approximately 2000 lux) at room temperature for 7-10 days and a large number of colonies with ampicillin resistance were obtained.

### (2-ii) Quantification of the antibody expressed in the transformants by means of ELISA

Each of the colonies grown on the plates were transferred to 2 ml of BG-11 liquid medium (containing 5 µg/ml ampicillin) and cultured under illumination (approximately 2000 lux) at room temperature for 10 days using a rotary shaker. This culture solution was transferred to 100 ml of BG-11 liquid medium (containing 50 µg/ml ampicillin) and cultured for two weeks, and then again transferred to three 100 ml BG-11 liquid solutions (each containing 50 µg ml ampicillin) and cultured for two weeks. These cultured cells were collected by centrifugation at 10000 rpm for 10 minutes, and suspended in 15 ml of PBS (5.75 g/l Na₂HPO₄, 1.0 g/l KH₂PO₄, 40 g/l sodium chloride and 1.0 g/l potassium chloride, pH 6.7-6.8) containing 1 mM PMSF. This cell suspension was distracted using an ultrasonic shredding equipment (Sonicator Model W-220: manufactured by Heat Systems-Ultrasonic, Inc.) (15 sec × 5) and centrifuged at 15000 rpm at 4°C for 20 minutes to obtain the supernatant. To the cell extract obtained, ammonium sulfate was added up to a saturation of 30 %, and the resulting salted out products were collected by centrifugation (at 15000 rpm at 4°C for 20 minutes) (0-30 % fraction). To the supernatant, ammonium sulfate was added up to a saturation of 80 %, and again salted out proteins were collected by centrifugation (at 15000 rpm at 4°C for 20 minutes) (30-80 % fraction). Each of the obtained 0-30 % fractions and 30-80 % fractions were dissolved in 5 ml PBS, and each of the solutions was placed into a dialysis tube and dialyzed overnight against 2 l of PBS at 4°C. Each fraction thus obtained was used to examine by use of Enzyme Linked Immunosorbent Assay (ELISA) according to the manner described below whether the antibody molecule was expressed in the transformed cyanobacteria cells and whether this antibody molecule had activity or not.

In each well of a 96-well plate (manufactured by Nunc Inc.), 50 µl of mouse monoclonal antibody Idio No. 33 (30 µg/l) or Idio No. 3 (30 µg/l) (which recognize the Idiotope of monoclonal antibody CLN-IgG; see Japanese Laid-Open Patent Publication No. 101999/1995 and U.S. Patent No. 5,589,573) were placed as primary antibody, and incubated at 37°C for 90 minutes. After discarding the primary antibody and washing each well (once) with 200 µl PBS containing 0.05 % Tween 20, each well was filled with 50 µl PBS containing 1 % BSA and incubated at 37°C for one hour. After incubation, 1% BSA was discarded and each well was filled with the two types of ammonium sulfate fractions which had been diluted with PBS containing 1 % BSA (for Idio No. 33, the fractions were diluted in 1/10, 1/100 or 1/200 and for Idio No. 3, the fractions were diluted in 1/5, 1/10 or 1/50). After incubation at 37°C for 30 minutes, the samples were discarded and each well was washed (twice) with 200 µl PBS containing 0.05 % Tween 20. Then each well was filled with 50 µl of peroxidase labeled anti-human IgG (manufactured by The Binding Site LTD Inc.) diluted in 1/2000 with PBS containing 0.05 % Tween 20, and then incubated at 37°C for 30 minutes. After discarding the secondary antibody and washing each well six times with 200 µl PBS containing 0.05 % Tween 20, each well was filled with 50 µl of an ortho-phenylenediamine solution (citric acid containing 12 mg/30 ml ortho-phenylenediamine and 6 µl/30 ml hydrogen peroxide) and incubated in the dark at 37°C for 30 minutes. Immediately after subjecting to reaction, each well was filled with 50 µl of 5 N sulfuric acid to terminate the reaction. Plates in which the reaction was completed were subjected to colorimetry at two wavelengths, 490 nm and 660 nm, using a microplate reader (manufactured by Corona Inc.; MTP-32). As a result, only in the 30-80 % ammonium sulfate fraction, reaction with mouse monoclonal antibodies Idio No. 33 and Idio No. 3 was found, which suggested that the antibody molecule expressed in the cyanobacteria had activity. Moreover, the amount of antibody CLN-H11 contained in the 30-80 % ammonium sulfate fraction obtained on the basis of this value, was approximately 1.35 µg (per 1 OD₂₈₀ₙₘ of the 30-80 % ammonium sulfate fraction). Judged from the total amount of soluble proteins of 88 units (OD₂₈₀ₙₘ), the amount expressed in cyanobacteria was estimated to be 0.24 µg/l OD₂₈₀ₙₘ unit.

The extract prepared from the cells which express CLN-IgG was passed through a Protein G Sepharose column (HiTrap™ protein G, manufactured by Pharmacia LKB Biotechnology Inc.) to bind the antibody molecule (CLN-IgG) to Protein G. After intensive washing of the column with PBS, the antibody molecule was eluted with 0.1 M glycin-HCl, 0.5 M sodium chloride (pH 2.7). The antibody solution obtained was 10-fold concentrated by use of UltraFree (M.W. 5000, manufactured by Millipore Inc.). and then its electro-chemical characteristics were compared with those of monoclonal antibody CLN-IgG obtained from the culture supernatant of human-human hybridoma CLN-H11 (ATCC HB 8307) (see, Japanese Patent Publication No. 59878/1989; U.S. Patent No. 4,618,577) by means of SDS-polyacrylamide gel electrophoresis and silver staining (manufactured by Nihon BioLad Inc.) according to the method of Laemmli (Nature. 227: 680, 1970). As a result, in the lane of the electrophoresed antibody solution obtained from the cyanobacteria extract, besides the band of the heavy chain, a band showing the same mobility as that of the light chain which does not bind to Protein A was detected.

The above results show that by simultaneous expression of an antibody heavy chain gene and light chain gene in cyanobacteria cells, it is possible to create *in vivo* an antibody which has activity.

## Claims

1. A method for preparing an antibody molecule characterized in that cyanobacteria cells are transformed with a carrier DNA which contains an antibody heavy chain gene or an antibody light chain gene to individually express an antibody heavy chain protein and an antibody light chain protein in the cyanobacteria, followed by associating the antibody heavy chain protein and the antibody light chain protein *in vitro*.

2. A method for preparing an antibody molecule characterized in that cyanobacteria cells are transformed with a carrier DNA which contains an antibody heavy chain gene and an antibody light chain gene to individually express an antibody heavy chain protein and an antibody light chain protein in the cyanobacteria, followed by associating them *in vivo*.

3. A method according to Claim 1 or Claim 2, wherein the antibody heavy chain gene and the antibody light chain gene are cDNA, RNA or synthetic DNA encoding the heavy chain and the light chain of human monoclonal antibody CLN-IgG.

4. A method according to Claim 1, wherein the carrier DNA which contains the antibody heavy chain gene or the antibody light chain gene contains an operon of the order (promoter region)-(DNA fragment containing a SD-like sequence)-(translation initiation codon, ATG)-(antibody heavy chain or light chain gene)-(translation termination codon)-(terminator region) which is expressible in the cyanobacteria cells.

5. A method according to Claim 2, wherein the carrier DNA which contains the antibody heavy chain gene and the antibody light chain gene contains an operon of the order (promoter region)-(DNA fragment containing a SD-like sequence)-(translation initiation codon, ATG)-(antibody heavy chain gene)-(translation termination codon)-(spacer region)-(DNA fragment containing a SD-like sequence)-(translation initiation codon)-(antibody light chain gene)-(translation termination codon)-(terminator region) which is expressible in the cyanobacteria cells.

6. A method according to Claim 4 or Claim 5, wherein the promoter region is the promoter region of ribulose-1,5-bisphosphate carboxylase / oxygenase gene, promoter I and II of Cyanophage N1, the promoter region of *psbA* gene of *Anabaena* PCC7120, or the promoter region of *RuBisCO* gene which can be prepared by cutting out plasmid pANE18 using restriction enzymes *Eco*RI, *Sac*I and *Pst*I according to a conventional method.

7. A method according to Claim 4 or Claim 5, wherein the SD-like sequence is GGAGG or GGAG.

8. A method according to Claim 4 or Claim 5, wherein the terminator region is the terminator region of *psbA* gene of *Anabaena* PCC7120, the terminator region of *SigA* gene, or the terminator region of *RuBisCO* gene which can be prepared by cutting out plasmid pANP1155 using restriction enzymes *Eco*RI and *Pso*I according to a conventional method.

9. A method according to Claim 5, wherein the spacer region is the spacer region which lies between a large subunit gene and a small subunit gene which are located in ribulose-1,5-bisphosphate carboxylase / oxygenase operon.

10. A method according to Claim 1 or Claim 2, wherein the cyanobacteria cells are *Anacystis nidulans* 6301 strain (*Synechococcus* PCC6301), *Anacystis nidulans* R2 strain (*Synechococcus* PCC7942), *Synechococcus* PCC7002, *Synechococcus* PCC7418 (*Aphanothece halophytica*), *Synechocystis* PCC6803, *Synechocystis* PCC6714, *Spirulina platensis*, *Anabaena* PCC7120 (*Nostoc* PCC7120). *Nostoc* PCC7119 (*Anabaena* PCC7119), or *Calothrix* PCC7601.

11. A method according to Claim 1, wherein the obtained antibody heavy chain protein and the antibody light chain protein are mixed at a ratio wherein amounts of the heavy chain protein and the light chain protein become almost the same, are incubated at a temperature of approximately 4-40°C, and are optionally dialyzed against a suitable buffer solution to associate the antibody heavy chain protein and the antibody light chain protein.

12. An antibody molecule obtained according to the method described in Claim 1 or Claim 2.
